# EUROPEAN PATENT SPECIFICATION

(11) **EP 0 447 739 B1**
(45) Date of publication and mention of the grant of the patent: **17.08.1994**
(21) Application number: 90830541.0
(22) Date of filing: 20.11.1990
(51) Int. Cl.: G01B 11/30, G01N 33/36

(54) **A method and a device for the objective evaluation of the crushing of pile fabrics, particularly velvets for covering motor vehicle seats**
Verfahren und Vorrichtung für das objektive Evaluieren des Zerdrückens von Plüschgeweben, insbesondere Velours in das Bedecken von Automobilsitzen
Méthode et dispositif pour l'évaluation objective de l'écrasement de tissus peluchés, en particulier du velours pour recouvrir les sièges d'un véhicule

(30) Priority: 20.12.1989 IT 6813789
(43) Date of publication of application: 25.09.1991
(73) Proprietor: FIAT AUTO S.p.A., 10135 Torino (IT)
(72) Inventor: Iulita, Piero, I-10137 Torino (IT)
(74) Representative: Gerbino, Angelo

(56) References cited:
- WO-A-84/00213
- US-A- 4 029 420
- PATENT ABSTRACTS OF JAPAN, vol. 11, no. 49 (P-547)[2496], 14th February 1987 & JP-A-61 217 742

## Description

The present invention relates to a method and a device for evaluating the crushing of fabrics, according respectively to the pre-characterising portions of the enclosed claims 1 and 7.

US-A-4 029 420 discloses a method and a device of the kind indicated above for measuring light reflectance from fabrics. This known device comprises a sensing area, wherein the fabric to be tested is located, three light sources for illuminating the sensing area and a photoelectric cell for detecting light reflected from the fabric in the sensing area.

More particularly the present invention relates to a method and a device for evaluating the crushing of pile fabrics, particularly velvets.

The need to make evaluations of this type results from the fact that a very important requisite for velvets, particularly those used to cover motor vehicle seats, is that they be resistant to crushing, that is, to flattening by compression. In fact, the presence of a passenger on the seat causes the pile of the velvet to be crushed and it regains its original appearance with difficulty. This causes a very unpleasant aesthetic effect since the colour of the crushed regions varies according to the angle from which it is seen (variability) and is different from that of the surrounding regions.

A known technique for evaluating- the crushing of pile fabrics is based on a visual examination carried out by several observers each of whom expresses a subjective judgement on the colour variation of a sample of fabric which has been subjected to different imprint pressures in an oven at a temperature of 60°. In particular, the minimum pressure at which the phenomenon arises is identified.

The known technique has a disadvantage that it is based on subjective elements which are therefore completely dependent on the different sensitivities of the observers, and this detracts from the necessary pre-cision and repeatability of the measurement.

The object of the present invention is to provide an objective method for evaluating the crushing of pile fabrics which enables the phenomenon to be quantified rigorously.

A first subject of the present invention is a method further comprising the features indicated in the characterising portion of the enclosed claim 1.

A further subject of the present invention is a device, which enables the method described above to be carried out, further comprising the features indicated in the characterising portion of the enclosed claim 1.

According to the invention, it is thus possible to assess objectively the proneness of the various fabrics to crushing with the use of repeatable numerical parameters.

Another advantage offered by the present invention lies in the fact that it enables a reduction in the personnel making the evaluation, since only one operator is required. The known technique, on the other hand, requires at least four or five observers in order to balance the subjective evaluation errors which they would make if they were acting individually.

A further advantage of the evaluation method according to the present invention consists of its greater sensitivity which enables it to evaluate colour variations, and hence crushing, which are not perceptible to the human eye.

Further advantages and characteristics of the present invention will become clear from the detailed description which follows with reference to the appended drawings, provided by way of non-limiting example, in which:
Figure 1 is a schematic view of a test device according to the invention, and
Figure 2 shows a detail of the device of Figure 1 on an enlarged scale.

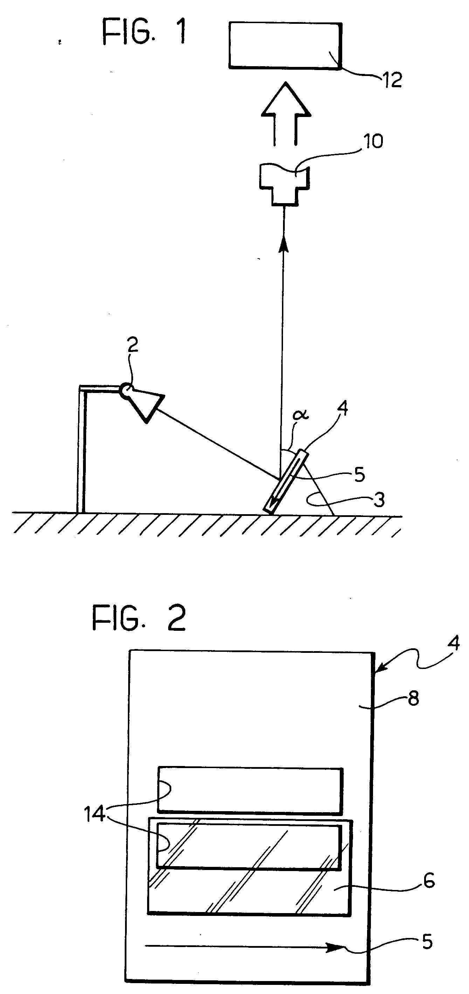

With reference to Figure 1, a test device for evaluating the crushing of pile fabrics, particularly velvets, comprises a light source 2 which illuminates uniformly a fabric sample 4 positioned on an orientable support 3. The sample 4, the direction of the pile of which is indicated by the arrow 5, has a first, crushed portion 6 and a second, uncrushed portion 8 (Figure 2).

The test device also includes a high-definition video camera 10 which provides digital images of the surface of the sample 4, and an image analyser 12 of known type, for example, of the IBAS type marketed by the company KONTRON.

In order to make the measurements, the samples 4 are crushed in an oven by pressures of different magnitudes. The weights used to generate the pressures are preferably rectangular in plan so that the boundary lines separating the crushed portions 6 and the uncrushed portions 8 are straight.

Each partially- crushed sample 4 is then removed from the oven and placed on the support means 3 in an orientation such that the light rays emitted by the light source 2 fall on it perpendicularly and the video camera 10 films it at an angle such that, because of the variability of the first, crushed portion 6, the colour difference between the first and second portions 6, 8 is greatest.

The digital images of the video camera 10 are then transmitted to the image analyser 12. This implements an evaluation program which constructs two parallel, equal, rectangular analysis windows 14 which face each other (Figure 2) and analyse the two portions 6, 8 of the sample 4 respectively. In order to increase the precision of the measurement, it is important for the boundary lines between the two portions 6, 8 to be straight and parallel to the edges of the analysis windows 14.

The evaluation program measures the average degree of greyness of the two portions 6, 8 and attributes to each a value between 0, which corresponds to black, and 255, which corresponds to white, possibly making corrections by comparison with the values obtained by analysing uncrushed samples.

The difference between the two average degrees of greyness thus obtained is adopted as an index of the crushing of the first portion 6 of the sample 4. The fact that the human eye is more sensitive to colour differences between two adjacent regions than to the absolute values of their coloration is thus taken into account.

The variation of the difference between the values, which is adopted as the index, with changes in the imprint pressure thus enables the behaviour of the fabric under examination when crushed to be described objectively without any subjective evaluation errors.

Finally, if the method described up to this point is repeated at different angles, it is possible to monitor the variation of the imprint index with changes in the angle of observation (the variability). It is thus possible to calculate a new parameter characteristic of the velvet under test. This parameter can be adopted as the "variation index".

According to other embodiments of the invention it is, for example, possible to vary the angle at which the rays from the light source 2 strike the sample 4, i.e. not perpendicularly. Obviously, however, if a homogeneous series of measurements is to be made, any changes to the test conditions must be kept constant once they have been implemented.

## Claims

1. A method for evaluating the crushing of fabrics, comprising the steps of:
- uniformly illuminating a sample of fabric (4) to be evaluated and a reference sample of fabric,
- producing an image of the surface of the illuminated samples,
- examining the image by means of an image analyser (12) which can evaluate the degree of reflectivity of the two samples,
said method being characterised in that:
- the sample to be evaluated and the reference sample are respectively a first (6) and a second (8) portion of the same fabric, which is a pile fabric, particularly a velvet,
- prior to illuminating the fabric (4), said first portion (6) is crushed by applied pressure, whereas said second portion (8) remains uncrushed,
- said image is a digital image, and
- after examining said image by means of an image analyser (12),the difference between the degree of reflectivity of the first (6) and the second (8) portions of the fabric (4) is adopted as an index of the crushing of the first portion (6).

2. A method according to Claim 1, characterised in that the image of the fabric sample (4) is produced by a high-definition video camera (10).

3. A method according to Claim 2, characterised in that it is repeated several times on the same sample (4), the orientation of the latter relative to the video camera (10) being varied in the successive iterations of the method, so as to enable the calculation of a variation index for the fabric, which corresponds to the variation of the difference between the values of the degrees of reflectivity of the crushed portion (6) and of the uncrushed portion (8) of the fabric in dependence of the orientation of the sample (4) relative to the video camera (10).

4. A method according to any one of the preceding claims, characterised in that the light rays which illuminate the sample (4) strike it perpendicularly.

5. A method according to any one of the preceding claims, characterised in that the first and second portions (6, 8) of the sample (4) are separated by a straight boundary lines.

6. A method according to any one of the preceding claims, characterised in that the sample (4) is subjected to crushing in an oven.

7. A device for evaluating the crushings of fabrics comprising :
- means for uniformly illuminating a sample of fabric (4) to be evaluated and a reference sample of a fabric,
- means for producing an image of the surface of the illuminated samples,
- means for examining the image by means of an image analyses (12) which can evaluate the degree of reflectivity of the two samples,
said device being characterised in that :
- the sample to be evaluated and the reference sample are respectively a first (6) and a second (8) portion of the same fabric, which is a pile fabric, particularly a velvet,
and by :
- support means (3) for supporting a fabric sample (4),
- crushing means for applying a pressure to the said first portion (6) of the fabric, but not to the said second portion (8),
- means for creating a digital image of the illuminated portions (6, 8) of the fabric,
- an image analyser for examining the said digital image, the difference between the degree of reflectivity of the first (6) and the second (8) portions of the fabric (4) being adopted as an index of the crushing of the first portion (6).

8. a device according to Claim 7 characterised in that said support means are orientable, and in that it comprises video camera means (10) which can frame the sample (4) supported by the support means (3) in order to generate an image of the sample (4).

9. A device according to Claim 7, characterised in that the video camera means (10) are of the high-definition type.

10. A device according to Claim 7 or Claim 8, characterised in that the video camera means (10) can generate a digital video signal.

## Patentansprüche

1. Verfahren zur Auswertung der Verdrückung von Stoffen, umfassend die Schritte:
- gleichmäßiges Beleuchten einer auszuwertenden Stoffprobe (4) und einer Bezugsstoffprobe,
- Erzeugen eines Bildes der Oberfläche der beleuchteten Proben,
- Untersuchen des Bildes mittels eines Bildanalysators (12), der den Grad des Reflexionsvermögens der zwei Proben auswerten kann,
wobei das Verfahren dadurch gekennzeichnet ist, daß:
- die auszuwertende Probe und die Bezugsprobe ein erster (6) bzw. ein zweiter (8) Abschnitt desselben Stoffes sind, welcher ein Florstoff, insbesondere ein Samt ist,
- vor der Beleuchtung des Stoffes (4) der erste Abschnitt (6) durch ausgeübten Druck verdrückt wird, wogegen der zweite Abschnitt (8) unverdrückt bleibt,
- das Bild ein digitales Bild ist und
- nach der Untersuchung des Bildes mittels eines Bildanalysators (12) die Differenz zwischen dem Grad des Reflexionsvermögens des ersten (6) und des zweiten (8) Abschnitts des Stoffes (4) als ein Maß der Verdrückung des ersten Abschnitts (6) genommen wird.

2. Verfahren nach Anspruch 1,
dadurch gekennzeichnet,
daß das Bild der Stoffprobe (4) mittels einer hochauflösenden Videokamera (10) erzeugt wird.

3. Verfahren nach Anspruch 2,
dadurch gekennzeichnet,
daß es mehrere Male an der gleichen Probe (4) wiederholt wird, wobei die Orientierung der Letzteren relativ zur Videokamera (10) in den aufeinanderfolgenden Wiederholungen des Verfahrens verändert wird, um die Berechnung eines Änderungsmaßes für den Stoff zu ermöglichen, welches der Änderung der Differenz zwischen den Werten der Grade des Reflexionsvermögens des verdrückten Abschnitts (6) und des unverdrückten Abschnitts (8) des Stoffes in Abhängigkeit von der Orientierung der Probe (4) relativ zur Videokamera (10) entspricht.

4. Verfahren nach einem der vorhergehenden Ansprüche,
dadurch gekennzeichnet,
daß die Lichtstrahlen, die die Probe (4) beleuchten, diese senkrecht treffen.

5. Verfahren nach einem der vorhergehenden Ansprüche,
dadurch gekennzeichnet,
daß der erste und zweite Abschnitt (6, 8) der Probe (4) durch gerade Grenzlinien getrennt sind.

6. Verfahren nach einem der vorhergehenden Ansprüche,
dadurch gekennzeichnet,
daß die Probe (4) in einem Ofen der Verdrückung unterworfen wird.

7. Vorrichtung zur Auswertung der Verdrückung von Stoffen, umfassend:
- Mittel zum gleichmäßigen Beleuchten einer auszuwertenden Stoffprobe (4) und einer Bezugsstoffprobe,
- Mittel zum Erzeugen eines Bildes der Oberfläche der beleuchteten Proben,
- Mittel zum Untersuchen des Bildes mittels eines Bildanalysators (12), der den Grad des Reflexionsvermögens der zwei Proben auswerten kann,
wobei die Vorrichtung dadurch gekennzeichnet ist, daß:
- die auszuwertende Probe und die Bezugsprobe ein erster (6) bzw. ein zweiter (8) Abschnitt desselben Stoffes sind, welcher ein Florstoff, insbesondere ein Samt, ist,
und durch:
- Haltemittel (3) zum Halten einer Stoffprobe (4),
- Verdrückungsmittel zum Ausüben eines Druckes auf den ersten Abschnitt (6) des Stoffes, jedoch nicht auf den zweiten Abschnitt (8),
- Mittel zum Erzeugen eines digitalen Bildes der beleuchteten Abschnitte (6, 8) des Stoffes,
- einen Bildanalysator zum Untersuchen des digitalen Bildes, wobei die Differenz zwischen dem Grad des Reflexionsvermögens des ersten (6) und des zweiten (8) Abschnitts des Stoffes (4) als ein Maß der Verdrückung des ersten Abschnitts (6) genommen wird.

8. Vorrichtung nach Anspruch 7,
dadurch gekennzeichnet,
daß die Haltemittel orientierbar sind und daß sie Videokameramittel (10) umfaßt, welche die von den Haltemitteln (3) gehaltene Probe (4) einfassen können, um ein Bild der Probe (4) zu generieren.

9. Vorrichtung nach Anspruch 7,
dadurch gekennzeichnet,
daß die Videokameramittel (10) vom hochauflösenden Typ sind.

10. Vorrichtung nach Anspruch 7 oder Anspruch 8,
dadurch gekennzeichnet,
daß die Videokameramittel (10) ein digitales Videosignal generieren können.

## Revendications

1. Procédé pour évaluer l'écrasement de tissus, comprenant les phases consistant à :
- éclairer uniformément un échantillon de tissu (4) à évaluer et un échantillon de référence du tissu,
- produire une image de la surface des échantillons éclairés,
- examiner l'image à l'aide d'un analyseur d'images (12) qui est capable d'évaluer le degré de réflectivité des deux échantillons,
ledit procédé étant caractérisé en ce que :
- l'échantillon à évaluer et l'échantillon de référence sont respectivement une première partie (6) et une deuxième partie (8) du même tissu, qui est un tissu à poil, en particulier un velours,
- avant d'éclairer le tissu (4), ladite première partie (6) est écrasée par une pression appliquée, tandis que ladite deuxième partie (8) reste non écrasée,
- ladite image est une image numérique, et
- après examen de ladite image au moyen d'un analyseur d'images (12), la différence entre les degrés de réflectivité de la première partie (6) et de la deuxième partie (8) de tissu (4) est prise pour indice de l'écrasement de la première partie (6).

2. Procédé selon la revendication 1, caractérisé en ce que l'image de l'échantillon de tissu (4) est produite par une caméra vidéo à haute définition (10).

3. Procédé selon la revendication 2, caractérisé en ce qu'il est répété plusieurs fois sur le même échantillon (4), l'orientation de celui-ci par rapport à la caméra vidéo (10) étant modifiée dans les itérations successives du procédé, de manière à permettre de calculer un indice de variation du tissu, indice qui correspond à la variation de la différence entre les valeurs des degrés de réflectivité de la partie écrasée (6) et de la partie non écrasée (8) du tissu en fonction de l'orientation de l'échantillon (4) par rapport à la caméra vidéo (10).

4. Procédé selon une quelconque des revendications précédentes, caractérisé en ce que les rayons lumineux qui éclairent l'échantillon (4) tombent perpendiculairement sur ce dernier.

5. Procédé selon une quelconque des revendications précédentes, caractérisé en ce que les premières et deuxième parties (6, 8) de l'échantillon (4) sont séparées par des lignes limites droites.

6. Procédé selon une quelconque des revendications précédentes, caractérisé en ce que l'échantillon (4) est soumis à un écrasement dans un four.

7. Dispositif pour évaluer l'écrasement d'un tissu, comprenant :
- des moyens servant à éclairer uniformément un échantillon de tissu (4) qu'il s'agit d'évaluer et un échantillon de référence d'un tissu,
- des moyens servant à produire une image de la surface des échantillons éclairés,
- des moyens servant à examiner l'image à l'aide d'un analyseur d'images (12) capable d'évaluer le degré de réflectivité des deux échantillons,
ledit dispositif étant caractérisé en ce que:
- l'échantillon à évaluer et l'échantillon de référence sont respectivement une première partie (6) et une deuxième partie (8) du même tissu, qui est un tissu à poil, en particulier un velours,
et par :
- des moyens supports (3) servant à supporter un échantillon de tissu (4),
- des moyens d'écrasement servant à appliquer une pression sur ladite première partie (6) du tissu mais non pas à ladite deuxième partie (8) du tissu,
- des moyens servant à créer une image numérique des parties éclairées (6, 8) du tissu,
- un analyseur d'images servant à examiner ladite image numérique, la différence de degré de réflectivité entre la première partie (6) et la deuxième partie (8) du tissu (4) étant prise pour indice d'écrasement de la première partie (6).

8. Dispositif selon la revendication 7, caractérisé en ce que lesdits moyens supports sont orientables et en ce qu'il comprend des moyens (10) du type caméra vidéo qui peuvent cadrer l'échantillon (4) supporté par les moyens supports (3) pour engendrer une image de l'échantillon (4).

9. Dispositif selon la revendication 7, caractérisé en ce que les moyens (10) formant caméra vidéo sont du type à haute définition.

10. Dispositif selon la revendication 7 ou la revendication 8, caractérisé en ce que les moyens (10) du type caméra vidéo peuvent engendrer un signal vidéo numérique.
